# FASCICULE DE BREVET EUROPEEN

(11) **EP 2 257 521 B1**
(45) Date de publication et mention de la délivrance du brevet: **22.04.2015**
(21) Numéro de dépôt: 09728473.1
(22) Date de dépôt: 13.03.2009
(51) Int. Cl.: C07C 209/48, C07C 211/09, C07D 211/12, C07D 213/133, C07D 213/08

(54) **PROCEDE DE FABRICATION DE METHYLPENTAMETHYLENE DIAMINE ET METHYLPIPERIDINE**
VERFAHREN ZUR HERSTELLUNG VON METHYLPENTAMETHYLEN-DIAMIN UND METHYLPIPERIDIN
METHOD FOR MAKING METHYLPENTAMETHYLENE DIAMINE AND METHYLPIPERIDINE

(30) Priorité: 31.03.2008 FR 0801750
(43) Date de publication de la demande: 08.12.2010
(73) Titulaire: Rhodia Opérations, 93306 Aubervilliers (FR)
(72) Inventeur: MARION, Philippe, 69390 Vernaison (FR)
(74) Mandataire: Chatelan, Florence Anne
(86) Numéro de dépôt international: PCT/EP2009/052966
(87) Numéro de publication internationale: WO 2009/121704

(56) Documents cités:
- WO-A2-2004/060833
- US-A- 4 521 602
- US-A- 4 885 391
- US-A- 5 105 015
- ANONYMOUS: "Hydrogenation of nitrites" RESEARCH DISCLOSURE, MASON PUBLICATIONS, HAMPSHIRE, GB, vol. 371, no. 12, 1 mars 1995 (1995-03-01), XP007120238 ISSN: 0374-4353

## Description

La présente invention concerne un procédé de fabrication de méthyl-2-pentaméthylène diamine et de méthyl-3-pipéridine.

Elle concerne un procédé de fabrication de méthyl-2-pentaméthylène diamine et méthyl-3-pipéridine par hydrogénation du méthyl-glutaronitrile.

Le méthyl-glutaronitrile est un composé obtenu conjointement à l'adiponitrile dans le procédé d'hydrocyanation du butadiène. Il est déjà connu de transformer ce méthyl-glutaronitrile en méthyl-pentaméthylène diamine (MPMD) par hydrogénation en présence de différents catalyseurs comme le cobalt de Raney ou le nickel de Raney. Des taux de transformation élevés du méthyl-glutaronitrile en MPMD ont été obtenus en utilisant du méthylglutaronitrile pur et en réalisant l'hydrogénation sous des pressions élevées comme cela est décrit dans les brevets français 2 306 202, 2 306 203.

Il a également été décrit des procédés d'hydrogénation du méthylglutaronitrile en un mélange de méthyl-2-pentaméthylène diamine (MPMD) et méthyl-3-pipéridine (MPP) avec des catalyseurs à base de nickel ou de nickel de Raney sous des pressions élevées d'hydrogène. De tels procédés ont notamment été décrits dans les brevets français 2 306 204, 2 306 205, 2 306 206, 2 306 207 et 2 306 208. Selon le solvant et/ou le catalyseur utilisé, la proportion de MPMD formée par rapport au MPP est variable. Comme pour les procédés décrits ci-dessus, ces procédés utilisent comme matière première un composé méthyl-glutaronitrile (MGN) pur. Il a également été proposé un procédé d'hydrogénation du MGN pur en MPMD et MPP en présence de cobalt de Raney dopé avec du chrome par le brevet US4885391. Cette réaction est avantageusement réalisée en présence d'eau. Le taux de transformation du MGN en produits valorisables, à savoir MPMD et MPP, est au maximum de 85% avec formation d'environ 10% de produits lourds non valorisables.

La méthyl-2-pentaméthylène diamine (MPMD) est utilisée principalement dans deux applications, l'une comme monomère pour la fabrication de polyamide, en remplacement total ou partiel de l'hexaméthylène diamine, l'autre comme matière première dans la fabrication de la β-picoline qui est elle-même un intermédiaire de synthèse pour fabriquer le nicotamide. Ce procédé est décrit dans la littérature par exemple dans l'article de S. Lanini, R. Prins publié dans Appl. Cat. A 1996 137 pp 287-306 sous le titre "synthesis of 3-picoline from MGN over supported noble metals catalysts".

Il a également été proposé de synthétiser la β-picoline par déshydrogénation d'un mélange de méthyl-2-pentaméthylène diamine et méthyl-3-pipéridine comme décrit dans le brevet US 4401819.

Par ailleurs, le méthyl-glutaronitrile est principalement produit dans le procédé de fabrication de l'adiponitrile, intermédiaire de synthèse pour la fabrication de l'hexaméthylène diamine et du caprolactame qui sont des monomères importants pour la production des polyamides.

Le méthyl-glutaronitrile est séparé de l'adiponitrile par distillation et récupéré sous forme d'une fraction de distillation. En fait, la fraction récupérée est un mélange de composés dinitriles comprenant majoritairement du méthylglutaronitrile et quelques pourcents d'éthyl-succinonitrile et d'adiponitrile.

Une distillation supplémentaire est nécessaire pour obtenir le méthylglutaronitrile pur, produit utilisé dans les procédés d'hydrogénation de l'art antérieur. Les essais décrits dans le brevet US 4 521 602 montrent que la transformation du MGN en Méthyl 3-pyridine est obtenue avec un rendement de 80% à partir du MGN pur et uniquement 42% avec un mélange de dinitriles.

En outre, les procédés de transformation du MGN en MPMD et MPP décrits dans la littérature utilisent des pressions élevées en hydrogène pour obtenir un pourcentage de transformation de MGN en MPMD+MPP supérieur à 90%. Souvent, la réaction d'hydrogénation est réalisée en milieu liquide.

Pour permettre d'abaisser le coût de fabrication de la β-picoline, il est important de chercher des solutions permettant de réaliser la transformation du MGN soit sous forme pure soit contenu dans le mélange de dinitriles avec des rendements de transformation du MGN en MPMD et MPP élevés, supérieurs à 90% et sous des conditions opératoires plus douces que celles décrites dans l'art antérieur.

Un des buts de la présente invention est de proposer un procédé de fabrication de méthyl-2-pentaméthylène diamine et méthyl-3-pipéridine à partir de méthyl-glutaronitrile pur ou en mélange avec d'autres nitriles avec des taux de transformation en MPMD + MPP élevés, ces deux composés étant valorisables dans des procédés avals de fabrication de composés chimiques importants tels que la β-picoline, comme indiqué ci-dessus.

A cet effet, l'invention propose un procédé de fabrication de méthyl-2-pentaméthylène diamine et méthyl-3-pipéridine par hydrogénation de méthylglutaronitrile, caractérisé en ce que l'hydrogénation est réalisée en présence d'un catalyseur comprenant du cobalt, et comme éléments dopants du chrome et du nickel, à une pression absolue d'hydrogène inférieure à 50 bar, de préférence, inférieure à 40 bar, avantageusement comprise entre 10 et 35 bar.

Selon une autre caractéristique de l'invention, l'hydrogénation est réalisée en présence d'un solvant choisi dans le groupe comprenant préférentiellement les alcools, l'eau ou un mélange de ceux-ci. Il est également possible d'utiliser comme solvant l'un des composés issus de la réaction d'hydrogénation (MPMD et/ou MPP) en association avec de l'eau.

Avantageusement, l'hydrogénation est réalisée en présence d'un composé minéral basique fort choisi dans le groupe comprenant les hydroxydes alcalins et l'hydroxyde d'ammonium. De préférence, le composé basique est l'hydroxyde de potassium ou l'ammoniaque.

L'hydrogénation peut être réalisée à une température comprise entre 60 et 160 °C, avantageusement entre 80 et 140°C.

Selon une nouvelle caractéristique de l'invention, le catalyseur comprend une quantité de cobalt exprimée en Co élémentaire comprise entre 85% environ et 98% environ du poids total du catalyseur, une quantité de nickel exprimée en Ni élémentaire comprise entre 0,1% environ et 4% environ en poids du catalyseur et une quantité de chrome exprimée en Cr élémentaire comprise entre 0,1% environ et 4% environ en poids du catalyseur.

Selon une caractéristique préférée de l'invention, le cobalt présent dans le catalyseur est un cobalt de Raney.

Le catalyseur de l'invention permet d'obtenir un taux de transformation du MGN en MPMD et MPP très élevé, proche de 100% et ainsi d'éviter la formation d'une quantité importante de composés lourds ou à point d'ébullition élevé, comme cela était obtenu avec les catalyseurs de l'état de la technique.

En outre, selon un autre avantage du procédé de l'invention utilisant un catalyseur de composition particulière, il est possible d'utiliser comme matière première à hydrogéner, un mélange de dinitriles comprenant du méthylglutaronitrile, de l'éthyl-succinonitrile et de l'adiponitrile. Ce mélange de dinitriles correspond avantageusement à la fraction de distillation produite dans le procédé de fabrication de l'adiponitrile par double hydrocyanation du butadiène, permettant de séparer les dinitriles ramifiés (méthyl-glutaronitrile, éthylsuccinonitrile) de l'adiponitrile.

Ce mélange de dinitriles a généralement la composition pondérale suivante :

| | | |
|---|---|---|
| Méthyl-glutaronitrile | : comprise entre | 70 % et 95 % |
| Ethyl-succinonitrile | : compris entre | 5 % et 30 % |
| Adiponitrile | : compris entre | 0 % et 10 % |

Ainsi, le procédé de l'invention permet d'obtenir un taux de transformation élevé du MGN contenu dans ce mélange en MPMD et MPP.

Selon une caractéristique préférentielle de l'invention, le mélange de dinitriles peut être enrichi en méthyl-glutaronitrile avant l'alimentation dans l'étape d'hydrogénation, par exemple par distillation.

La méthyl-2-pentaméthylène diamine, la méthyl-3- pipéridine ou leurs mélanges peuvent être utilisés comme matières premières dans la fabrication de la β-picoline selon les procédés décrits dans les documents déjà cités précédemment.

Dans le mode de réalisation de l'invention utilisant comme matières premières un mélange de dinitriles, il est possible d'utiliser les composés issus de l'hydrogénation directement dans le procédé de fabrication de la β-picoline, sans séparation et purification de la MPMD et MPP. Toutefois, il peut être préférable de réaliser une séparation de ces deux composés des autres produits issus de l'hydrogénation de l'ESN ou de l'AdN. Cette séparation peut être réalisée par toutes les techniques connues et notamment la distillation.

D'autres avantages, détails de l'invention seront illustrés par les exemples donnés ci-dessous uniquement à titre illustratif.

### Exemple 1 comparatif (MGN pur):

2 g de catalyseur à base de Nickel de Raney comprenant 91 % en poids de Nickel, dopé avec 2% en poids de Cr sont ajoutés dans 100g d'une solution de MGN (pureté 99%) à 20%pds dans l'éthanol et 1,5g de NH₃ sous forme d'une solution aqueuse de NH₄OH à 28%pds de NH₃. Le réacteur est purgé à l'azote puis à l'hydrogène. Le milieu réactionnel est chauffé sous pression d'hydrogène, et agité quand la température désirée est atteinte (100°C). Le réacteur est refroidi pour maintenir la température du milieu à 100°C environ. La pression relative dans le réacteur est maintenue constante à une valeur de 25 bar. Quand l'hydrogène n'est plus consommé, marque de la fin de la réaction, un échantillon du milieu réactionnel est prélevé pour analyse par chromatographie en phase gaz.

Les résultats de l'analyse montrent que la conversion du MGN est totale et la sélectivité en MPMD est de 23%, celle en MPP de 44%. La somme des sélectivités en produits valorisables est donc de 67%.

### Exemple 2 de l'invention (MGN pur):

2 g de catalyseur à base de Cobalt de Raney comprenant 92% en poids de Cobalt, dopé avec 2,2% en poids de Cr et 2,4% en poids de Ni sont ajoutés dans 100g d'une solution de MGN (pureté 99%) à 20%pds dans l'éthanol et 1,5g de NH₃ sous forme d'une solution aqueuse de NH₄OH à 28%pds de NH₃. Le réacteur est purgé à l'azote puis à l'hydrogène. Le milieu réactionnel est chauffé sous pression d'hydrogène, et agité quand la température désirée est atteinte (100°C). Le réacteur est refroidi pour maintenir la température du milieu à 100°C environ. La pression dans le réacteur est maintenue constante à une valeur de 25bar. Quand l'hydrogène n'est plus consommé, marque de la fin de la réaction, un échantillon du milieu réactionnel est prélevé pour analyse par chromatographie en phase gaz.

Les résultats de l'analyse montrent que la conversion du MGN est totale et la sélectivité en MPMD est de 80%, celle en MPP de 20%. La somme des sélectivités en produits valorisables est donc de 100%.

Le taux de transformation du MGN en MPMD et MPP est notablement plus élevé que celui obtenu dans l'exemple 1 avec un catalyseur à base de Nickel de Raney mais également à celui indiqué dans le brevet US4885391. En effet, ce document décrit l'hydrogénation du MGN en MPMD et MPP en présence d'un catalyseur Cobalt de Raney dopé chrome. Le taux de transformation maximum est de l'ordre de 80 à 85% avec une formation de composés de haut point d'ébullition représentant environ 10% des produits obtenus.

### Exemple 3 comparatif (Mélange de dinitriles):

2 g de catalyseur à base de Nickel de Raney comprenant 91 % en poids de Nickel, dopé avec 2% en poids de Cr sont ajoutés dans 100g d'une solution à 20%pds dans l'éthanol d'un mélange de dinitriles appelé MGN brut comprenant :
86,9 % en poids de méthylglutaronitrile
11,2 % en poids d'éthylsuccinonitrile
1,9 % en poids d'adiponitrile
et 1,5g de NH₃ sous forme d'une solution aqueuse de NH₄OH à 28%pds de NH₃. Le réacteur est purgé à l'azote puis à l'hydrogène. Le milieu réactionnel est chauffé sous pression d'hydrogène, et agité quand la température désirée est atteinte (100°C). Le réacteur est refroidi pour maintenir la température du milieu à 100°C environ. La pression dans le réacteur est maintenue constante à une valeur de 25bar. Quand l'hydrogène n'est plus consommé, marque de la fin de la réaction, un échantillon du milieu réactionnel est prélevé pour analyse par chromatographie en phase gaz.

Les résultats de l'analyse montrent que la conversion du MGN est de 99,9% et la sélectivité en MPMD est de 22%, celle en MPP de 37%. La somme des sélectivités en produits valorisables est donc de 59 %.

### Exemple 4 de l'invention (Mélange de dinitriles):

2 g de catalyseur à base de Cobalt de Raney comprenant 92% en poids de Cobalt, dopé avec 2,2% en poids de Cr et 2,4% en Ni sont ajoutés dans 100g d'une solution à 20%pds dans l'éthanol d'un mélange de dinitriles appelé MGN brut comprenant :
86,9 % en poids de méthylglutaronitrile
11,2 % en poids d'éthylsuccinonitrile
1,9 % en poids d'adiponitrile
et 1,5g de NH₃ sous forme d'une solution aqueuse de NH₄OH à 28%pds de NH₃. Le réacteur est purgé à l'azote puis à l'hydrogène. Le milieu réactionnel est chauffé sous pression d'hydrogène, et agité quand la température désirée est atteinte (100°C). Le réacteur est refroidi pour maintenir la température du milieu à 100°C environ. La pression dans le réacteur est maintenue constante à une valeur de 25bar. Quand l'hydrogène n'est plus consommé, marque de la fin de la réaction, un échantillon du milieu réactionnel est prélevé pour analyse par chromatographie en phase gaz.

Les résultats de l'analyse montrent que la conversion du MGN est totale et la sélectivité en MPMD est de 58%, celle en MPP de 31%. La somme des sélectivités en produits valorisables est donc de 89 %.

### Exemple 5 de l'invention (Mélange de dinitriles):

2 g de catalyseur à base de Cobalt de Raney comprenant 92% en poids de Cobalt, dopé avec 2,2% en poids de Cr et 2,4% en Ni sont ajoutés dans 100g d'une solution à 20%pds dans l'éthanol d'un mélange de dinitriles appelé MGN brut comprenant :
86,9 % en poids de méthylglutaronitrile
11,2 % en poids d'éthylsuccinonitrile
1,9 % en poids d'adiponitrile
et 1,5g de NH₃ sous forme d'une solution aqueuse de NH₄OH à 28%pds de NH₃. Le réacteur est purgé à l'azote puis à l'hydrogène. Le milieu réactionnel est chauffé sous pression d'hydrogène, et agité quand la température désirée est atteinte (140°C). Le réacteur est refroidi pour maintenir la température du milieu à 140°C environ. La pression dans le réacteur est maintenue constante à une valeur de 25bar. Quand l'hydrogène n'est plus consommé, marque de la fin de la réaction, un échantillon du milieu réactionnel est prélevé pour analyse par chromatographie en phase gaz.

Les résultats de l'analyse montrent que la conversion du MGN est totale et la sélectivité en MPMD est de 64%, celle en MPP de 28%. La somme des sélectivités en produits valorisables est donc de 92 %.

### Exemple 6 de l'invention (Mélange de dinitriles):

2 g de catalyseur à base de Cobalt de Raney comprenant 92% en poids de Cobalt, dopé avec 2,2% en poids de Cr et 2,4% en Ni sont ajoutés dans 100g d'une solution à 20%pds dans un mélange MPP(méthyl-3-pipéridine)/eau 95/5 (parties en poids), d'un mélange de dinitriles appelé MGN brut comprenant :
86,9 % en poids de méthylglutaronitrile
11,2 % en poids d'éthylsuccinonitrile
1,9 % en poids d'adiponitrile
et 1,5g de NH₃ sous forme d'une solution aqueuse de NH₄OH à 28% pds de NH₃. Le réacteur est purgé à l'azote puis à l'hydrogène. Le milieu réactionnel est chauffé sous pression d'hydrogène, et agité quand la température désirée est atteinte (100°C). Le réacteur est refroidi pour maintenir la température du milieu à 100°C environ. La pression dans le réacteur est maintenue constante à une valeur de 25bar. Quand l'hydrogène n'est plus consommé, marque de la fin de la réaction, un échantillon du milieu réactionnel est prélevé pour analyse par chromatographie en phase gaz.

Les résultats de l'analyse montrent que la conversion du MGN est totale et la sélectivité en MPMD est de 49%, celle en MPP de 19%. La somme des sélectivités en produits valorisables est donc de 68%.

### Exemple 7 comparatif (Mélange de dinitriles):

2 g de catalyseur à base de Nickel de Raney comprenant 91% en poids de Nickel, dopé avec 2% en poids de Cr sont ajoutés dans 100g d'une solution à 20%pds dans un mélange MPP(méthyl-3- pipéridine)/eau 95/5 (parties en poids), d'un mélange de dinitriles appelé MGN brut comprenant :
86,9 % en poids de méthylglutaronitrile
11,2 % en poids d'éthylsuccinonitrile
1,9 % en poids d'adiponitrile
et 1,5g de NH₃ sous forme d'une solution aqueuse de NH₄OH à 28% pds de NH₃. Le réacteur est purgé à l'azote puis à l'hydrogène. Le milieu réactionnel est chauffé sous pression d'hydrogène, et agité quand la température désirée est atteinte (100°C). Le réacteur est refroidi pour maintenir la température du milieu à 100°C environ. La pression dans le réacteur est maintenue constante à une valeur de 25 bar. Quand l'hydrogène n'est plus consommé, marque de la fin de la réaction, un échantillon du milieu réactionnel est prélevé pour analyse par chromatographie en phase gaz.

Les résultats de l'analyse montrent que la conversion du MGN est totale et la sélectivité en MPMD est de 20%, celle en MPP de 13%. La somme des sélectivités en produits valorisables est donc de 33%.

## Revendications

1. Procédé de fabrication de méthyl-2-pentaméthylène diamine et méthyl-3-pipéridine par hydrogénation de méthyl-glutaronitrile, **caractérisé en ce que** l'hydrogénation est réalisée en présence d'un catalyseur comprenant du cobalt et comme éléments dopants du nickel et du chrome, à une pression absolue d'hydrogène inférieure à 50 bar.

2. Procédé selon la revendication 1, **caractérisé en ce que** la réaction d'hydrogénation est réalisée sous une pression absolue d'hydrogène comprise entre 10 et 35 bar.

3. Procédé selon la revendication 1 ou 2, **caractérisé en ce que** l'hydrogénation est réalisée en présence d'un solvant choisi dans le groupe comprenant les alcools, l'eau ou un mélange de ceux-ci.

4. Procédé selon la revendication 1, 2 ou 3, **caractérisé en ce que** l'hydrogénation est réalisée en présence d'un composé minéral basique fort choisi dans le groupe comprenant les hydroxydes alcalins et l'hydroxyde d'ammonium.

5. Procédé selon l'une des revendications précédentes en ce que l'hydrogénation est réalisée à une température comprise entre 60 et 160 °C.

6. Procédé selon l'une des revendications précédentes, **caractérisé en ce que** le pourcentage pondérale de cobalt dans le catalyseur, exprimé en Co est compris entre 85% et 98 % par rapport au poids de catalyseur

7. Procédé selon l'une des revendications précédentes, **caractérisé en ce que** le pourcentage pondérale de nickel dans le catalyseur, exprimé en Ni est compris entre 0,1 % et 4 % par rapport au poids de catalyseur.

8. Procédé selon l'une des revendications précédentes, **caractérisé en ce que** le pourcentage pondérale de chrome dans le catalyseur, exprimé en Cr est compris 0,1 % et 4 % par rapport au poids de catalyseur.

9. Procédé selon l'une des revendications précédentes, **caractérisé en ce que** le cobalt présent dans le catalyseur est un Cobalt de Raney.

10. Procédé selon l'une des revendications précédentes, **caractérisé en ce qu'**on introduit dans l'étape d'hydrogénation un mélange de dinitriles comprenant du méthyl-glutaronitrile, de l'éthyl-succinonitrile et de l'adiponitrile.

11. Procédé selon la revendication 10, **caractérisé en ce que** le mélange de dinitriles à la composition pondérale suivante :
| | | |
|---|---|---|
| Méthyl-glutaronitrile | : comprise entre | 70 % et 95 % |
| Ethyl-succinonitrile | : compris entre | 5 % et 30 % |
| Adiponitrile | : compris entre | 0 % et 10 % |

12. Procédé selon l'une des revendications 10 à 11, **caractérisé en ce que** le mélange de dinitriles est enrichi en méthyl-glutaronitrile avant l'alimentation dans l'étape d'hydrogénation.

## Patentansprüche

1. Verfahren zur Herstellung von 2-Methylpentamethylendiamin und 3-Methylpiperidin durch Hydrierung von Methylglutaronitril, **dadurch gekennzeichnet, dass** die Hydrierung in Gegenwart eines Katalysators, der Cobalt und als Dotierungselemente Nickel und Chrom umfasst, bei einem Wasserstoff-Absolutdruck von weniger als 50 bar durchgeführt wird.

2. Verfahren nach Anspruch 1, **dadurch gekennzeichnet, dass** die Hydrierungsreaktion unter einem Wasserstoff-Absolutdruck zwischen 10 und 35 bar durchgeführt wird.

3. Verfahren nach Anspruch 1 oder 2, **dadurch gekennzeichnet, dass** die Hydrierung in Gegenwart eines Lösungsmittels aus der Gruppe umfassend Alkohole, Wasser oder eine Mischung davon durchgeführt wird.

4. Verfahren nach Anspruch 1, 2 oder 3, **dadurch gekennzeichnet, dass** die Hydrierung in Gegenwart einer stark basischen anorganischen Verbindung aus der Gruppe umfassend Alkalihydroxide und Ammoniumhydroxid durchgeführt wird.

5. Verfahren nach einem der vorhergehenden Ansprüche, **dadurch gekennzeichnet, dass** die Hydrierung bei einer Temperatur zwischen 60 und 160°C durchgeführt wird.

6. Verfahren nach einem der vorhergehenden Ansprüche, **dadurch gekennzeichnet, dass** der Gewichtsprozentanteil von Cobalt im Katalysator, ausgedrückt als Co, zwischen 85% und 98%, bezogen auf das Gewicht des Katalysators, liegt.

7. Verfahren nach einem der vorhergehenden Ansprüche, **dadurch gekennzeichnet, dass** der Gewichtsprozentanteil von Nickel im Katalysator, ausgedrückt als Ni, zwischen 0,1% und 4%, bezogen auf das Gewicht des Katalysators, liegt.

8. Verfahren nach einem der vorhergehenden Ansprüche, **dadurch gekennzeichnet, dass** der Gewichtsprozentanteil von Chrom im Katalysator, ausgedrückt als Cr, zwischen 0,1% und 4%, bezogen auf das Gewicht des Katalysators, liegt.

9. Verfahren nach einem der vorhergehenden Ansprüche, **dadurch gekennzeichnet, dass** es sich bei dem im Katalysator vorliegenden Cobalt um Raney-Cobalt handelt.

10. Verfahren nach einem der vorhergehenden Ansprüche, **dadurch gekennzeichnet, dass** man im Hydrierungsschritt ein Gemisch von Dinitrilen, das Methylglutaronitril, Ethylsuccinonitril und Adiponitril umfasst, einträgt.

11. Verfahren nach Anspruch 10, **dadurch gekennzeichnet, dass** das Gemisch von Dinitrilen die folgende gewichtsbezogene Zusammensetzung aufweist:
Methylglutaronitril: zwischen 70% und 95%
Ethylsuccinonitril: zwischen 5% und 30%
Adiponitril: zwischen 0% und 10%.

12. Verfahren nach einem der Ansprüche 10 bis 11, **dadurch gekennzeichnet, dass** das Gemisch von Dinitrilen vor der Zuführung zum Hydrierungsschritt an Methylglutaronitril angereichert wird.

## Claims

1. Process for the manufacture of 2-methylpentamethylenediamine and 3-methylpiperidine by hydrogenation of methylglutaronitrile, **characterized in that** the hydrogenation is carried out in the presence of a catalyst comprising cobalt and, as doping elements, nickel and chromium at an absolute hydrogen pressure of less than 50 bar.

2. Process according to Claim 1, **characterized in that** the hydrogenation reaction is carried out under an absolute hydrogen pressure of between 10 and 35 bar.

3. Process according to Claim 1 or 2, **characterized in that** the hydrogenation is carried out in the presence of a solvent chosen from the group consisting of alcohols, water or a mixture of these.

4. Process according to Claim 1, 2 or 3, **characterized in that** the hydrogenation is carried out in the presence of a strong basic inorganic compound chosen from the group consisting of alkali metal hydroxides and ammonium hydroxide.

5. Process according to one of the preceding claims, **characterized in that** the hydrogenation is carried out at a temperature of between 60 and 160°C.

6. Process according to one of the preceding claims, **characterized in that** the percentage by weight of cobalt in the catalyst, expressed as Co, is between 85% and 98%, with respect to the weight of catalyst.

7. Process according to one of the preceding claims, **characterized in that** the percentage by weight of nickel in the catalyst, expressed as Ni, is between 0.1% and 4%, with respect to the weight of catalyst.

8. Process according to one of the preceding claims, **characterized in that** the percentage by weight of chromium in the catalyst, expressed as Cr, is between 0.1% and 4%, with respect to the weight of catalyst.

9. Process according to one of the preceding claims, **characterized in that** the cobalt present in the catalyst is a Raney cobalt.

10. Process according to one of the preceding claims, **characterized in that** a mixture of dinitriles comprising methylglutaronitrile, ethylsuccinonitrile and adiponitrile is introduced into the hydrogenation stage.

11. Process according to Claim 10, **characterized in that** the mixture of dinitriles has the following composition by weight:
Methylglutaronitrile : of between 70% and 95%
Ethylsuccinonitrile : of between 5% and 30%
Adiponitrile : of between 0% and 10%.

12. Process according to either of Claims 10 and 11, **characterized in that** the mixture of dinitriles is enriched in methylglutaronitrile before feeding to the hydrogenation stage.
